# EUROPEAN PATENT SPECIFICATION

(11) **EP 1 710 586 B1**
(45) Date of publication and mention of the grant of the patent: **11.08.2010**
(21) Application number: 06014487.0
(22) Date of filing: 09.10.2001
(51) Int. Cl.: G01N 33/543, G01N 33/58, G01N 33/558

(54) **Immunological analytical device for the determination of advanced glycosylation end products (AGEs)**
Immunologisch analytische Test-Vorrichtung zur Bestimmung von AGEs (advanced glycosylation end products)
Dispositif analytique immunologique pour détermination du produits terminaux d`une glycosylation avancée (AGEs)

(30) Priority: 09.10.2000 CN 00253607 U
(43) Date of publication of application: 11.10.2006
(62) Divisional of application: 01124678.2
(73) Proprietor: Chan, Wing-yee, Taipei, Taiwan (CN); Liu, Yung-Hsiang, Taipei, Taiwan (CN)
(72) Inventor: Chan, Wing-yee, Taipei, Taiwan (CN); Liu, Yung-Hsiang, Taipei, Taiwan (CN)
(74) Representative: Kador & Partner

(56) References cited:
- EP-A- 0 291 194
- WO-A-97/34147
- US-A- 5 160 486
- US-A- 5 670 381

## Description

### BACKGROUND OF THE INVENTION

### 1. Field of the invention

The invention relates to an immunological analytical method, reagents and devices for the determination of the glycosylated protein antigen or anti-glycosylated protein antibody, which determine the immunological reaction of the glycosylated protein antigen or anti-glycosylated protein antibody by agglutination phenomenon or accompanied changes of absorbance or color.

### 2. Description of the related art

The pathogenic mechanism of diabetes has been thoroughly understood today. For example, it is know that, under the situation of hyperglycemia, a protein (e.g., albumin) might be glycosylated into glycosylated protein (e.g., Advanced Glycosylated End Products (AGEs)), and this would cause abnormal destruction of cells, particular in a diabetic patient. Further, while the blood sugar level of a diabetic patient can currently be controlled effectively with drugs, the efficient manipulation of complicated condition through earlier prediction is still impossible. Therefore, it has been devoted to develop a specific approach or agent to determine whether the AGE of the diabetes is present or not such that the attending practitioner could prevent the occurrence of the complicated condition in a diabetic patient at the earliest stage, or block the progression of the complicated condition. Among such specific approach or reagent for determining the AGE of the diabetes, the most specific and sensitive one comprises of determining the glycosylated protein antigen or anti-glycosylated protein antibody, such as, for example, the advanced glycosylated end products, by immunological analytic techniques. However, no immunological analytic technique for determining the glycosylated protein antigen or anti-glycosylated protein antibody is available at present.

In view of the forgoing, the inventor of this application has been studied extensively and finally, developed an immunological analytic technique or devices for determining the glycosylated protein antigen or anti-glycosylated protein antibody, which essentially comprise of determining the immunological reaction of the glycosylated protein antigen or anti-glycosylated protein antibody by agglutination phenomenon or accompanied changes of absorbance or color, and the invention is thus accomplished.

### SUMMARY OF THE INVENTION

We describe an immunological analytic technique for determining the glycosylated protein antigen (antibody) with the glycosylated protein antibody (antigen), which comprises of determining whether any glycosylated protein antigen or antibody is present or not in the sample based on the agglutination phenomenon or accompanied changes of absorbance or color by immunological analytical technique.

Furthermore, we describe an immunological analytical reagent for determining the glycosylated protein antigen (antibody) with the glycosylated protein antibody (antigen), which can determine whether any glycosylated protein antigen or antibody is present or not in the sample based on the agglutination phenomenon due to the formation of the immnological complex between, for example, the anti-glycosylated protein antibody and the glycosylated antigen (e.g., Advanced Glycosylated End Products (AGEs) antigen).

We describe an immunological analytical reagent for determining the glycosylated protein antigen (antibody) with the glycosylated protein antibody (antigen), which can determine whether any glycosylated protein antigen or antibody is present or not in the sample based on the change of absorbance due to the formation of the immunological complex between, for example, the anti-glycosylated protein antibody and the glycosylated antigen (e.g., Advanced Glycosylated End Products (AGEs) antigen).

The invention provides an immunological analytical test strip for determining the glycosylated protein antigen (antibody) with the glycosylated protein antibody (antigen), which can determine whether any glycosylated protein antigen or antibody is present or not in the sample based on the color change against the reference line due to the formation of the immunological complex between, for example, the anti-glycosylated protein antibody and the glycosylated antigen (e.g., Advanced Glycosylated End Products (AGEs) antigen) on a base plate as defined in the claims.

The features, objectives and advantages of the invention will become apparent from perusal of the following description with reference to the appended figures in which:
Figure 1 is the three-dimensional structural view of the immunological chromatographic test strip according to the invention;
Figure 2 is the three-dimensional outlined view of the box of a waterproof device, which box is used for accommodating the immunological chromatographic test strip shown in Figure 1; and
Figure 3 is the overall exploded schematic view of the box of the waterproof device shown in Figure 2, accommodating the test strip according to the invention.

### Description of Symbols:

- 10: Test strip
- 11: Water-absorption pad
- 12: Displaying carrier fiber block
- 13: Porous fiber membrane
- 14: Reading zone
- 15: Reference zone
- 16: Absorption pad
- 17: Base plate
- 18: The front end of the test strip 10
- 19: The rear end of the test strip 10
- 20: Waterproof device box
- 21: Sample port
- 22: Port of the reaction zone

### DETAILED DESCRIPTION OF THE PREFERRED EMBODIMENT

As described above, the invention relates to the determination of the presence or not of a glycosylated protein, such as the advanced glycosylated end products in a sample to be tested by several immunological analytical techniques. Antibody used in the invention can be those raised immunologically in rabbit or goat with purified antigen (e.g., AGE antigen), or those obtained as hybridoma by immunizing mice (e.g., monoclonal antibody).

Immunological analytical techniques are known in the art. For example, Fujikawa H., and Igarashi, H.(Appl. Envir. Microbiol., 54/10, 2345-2348, 1998) disclosed flash emulsion agglutination reagents formed from high density latex granules for detecting enterotoxin A~E of Staphylococcus. Delanghe, J. R., Chapele, J. P., and Vander schueren, S. C., proposed a colorimetric method for detecting myoglobin (Clin. Chem., 36/9, 1675-1678, 1990). WO 88/08534 (1988) disclosed an immunological analytical device comprising an immunological chromatographic membrane as the medium for determining HCG and LH. US Patent No. 5,236,652 (1993) disclosed an immunological chromatographic technique for determining non-protein antigen.

None of the above-mentioned techniques taught about the immunological analytical technique and device for determining the glycosylated protein antigen or anti-glycosylated protein antibody disclosed by the invention.

We describe an immunological analytic reagent for determining the glycosylated protein antigen (antibody) with the glycosylated protein antibody (antigen), which comprises of determining whether any glycosylated protein antigen or antibody (e.g., Advanced Glycosylated End Products (AGEs) antigen) is present or not in the sample based on an immunological agglutination technique, which comprises:
A displaying carrier emulsion, and the glycosylated protein antigen or anti-glycosylated protein antibody immobilized on the surface of said displaying carrier; which, after contacting said emulsion reagent with a sample to be tested, can determine whether a glycosylated protein antigen or antibody is present or not based on the resultant agglutination phenomenon.

In one experiment, one or more glycosylated protein antibody is immobilized on said displaying carrier in the emulsion, and a blocking protein is used to fill fully voids in the carrier. When a test sample containing a given amount of glycosylated protein antigen (e.g., Advanced Glycosylated End Products (AGEs) antigen) mixes and reacts with a displaying carrier emulsion comprising one or more immobilized antibody on the reacting plate, immunological complexes as "displaying carrier-antibody, or multiple antibodies-AGEs-antibody, or multiple antibodies-displaying carrier....etc.". Such reaction will result into a visible agglutination as a positive response within 3-5 minutes. On the other hand, if no AGE antigen present in the test sample, after mixing with the displaying carrier emulsion comprising one or more immobilized antibody, no agglutination will occur therebetween, and hence is considered as a negative response.

The displaying carrier used in the invention is a fine colored particulate with a particle size of 0.01 - 60 micron. Such particulate may be any of latex micro-particles, dye micro-particles, liposomes, colloidal gold particles, carbon black micro-particles, and the like.

The term "antibody" as used herein refers to one or more of the above-mentioned anti-glycosylated protein antibodies. Immobilization of a glycosylated protein antigen having one structure or a glycosylated protein antigen having various structures can be used to determine whether any antibody of such glycosylated protein antigen is present in the test sample or not; if no agglutination, it is a negative reaction; on the other hand, agglutination means positive reaction.

We describe an immunological analytic reagent for determining the glycosylated protein antigen (glycosylated protein antibody) with the glycosylated protein antibody (glycosylated protein antigen), which comprises of detecting the glycosylated protein antigen or anti-glycosylated protein antibody (e.g., Advanced Glycosylated End Products (AGEs) antigen or antibody thereof) based on an immunological turbidimetric technique, which comprises:
A displaying carrier suspension, immobilized on the surface of said displaying carrier with the above-mentioned glycosylated protein antigen or antibody; and
A device for measuring absorbance;
which, after contacting said reagent with a sample to be tested, can determine whether a glycosylated protein antigen or antibody is present or not based on the resultant change or not of absorbance.

In one experiment, one or more glycosylated protein antibody is immobilized on said displaying carrier in the emulsion, and a blocking protein is used to fill fully voids in the carrier. A negative serum standard, a weak positive serum standard, and a unknown test sample are prepared, respectively. To three colorimetric tubes A1, A2 and A3 are charged each of 250 micro-liter of displaying carrier emulsion containing immobilized one or more antibodies. Then, adds 20 micro-liter of the negative serum standard into the colorimetric tube A1; adds 20 micro-liter of the weak positive serum standard into the colorimetric tube A2; and adds 20 micro-liter of the unknown sample into the colorimetric tube A3. Immediately after the additions are accomplished, the tubes are subjected to turbidity or absorbance measurement at 340 nm, and recorded the respective optical density (OD) value. At 240 seconds after addition of the sample, the respective OD values are recorded again. The difference by subtracting the first recorded OD values from the second recorded OD values, respectively, is the OD value of the immunological reaction. By comparing three OD differences thus obtained, when the difference of OD value from the unknown sample is greater than that from the weak positive serum standard, it can be considered as a positive response. On the other hand, when the difference of OD value from the unknown sample is less than that from the weak positive serum standard, it can be considered as a negative response. Furthermore, by preparing a series of standard solutions each having a know concentration, such as, for example, 0, 1, 2, 4, 8, and 16 units/ml, the concentration in the test sample can be calculated from the standard curve plotted from data obtained with such standard solutions.

The displaying carrier used herein is a fine particulate or enzyme. Such particulates may be any of latex micro-particles, liposomes, polyethylene glycol micro-particulates, NAD micro-particles, carbon micro-particles, dye micro-particles, enzyme, NADH micro-particles, and the like with a particle size of 0.001 - 20 micron and a spectrophotometric range of 260 nm to 840 nm.

The term "antibody" as used herein refers to one or more of the above-mentioned anti-glycosylated protein antibodies. Immobilization of a glycosylated protein antigen having one structure or a glycosylated protein antigen having various structures can be used to determine whether any antibody of such glycosylated protein antigen is present in the test sample or not. Alternatively, a competitive method can used to yield a same experimental result. For example, when conducted in the same manner as described above, additionally, a given amount of glycosylated protein antigen or antibody with known concentration can be used together with each of the test sample, the positive standard solution and the negative standard solution to react respectively the displaying carrier emulsion having the antibody or antigen immobilized thereon, a similar result can be obtained as above.

The invention provides an immunological analytical test strip for determining the glycosylated protein antigen (antibody) with the glycosylated protein antibody (antigen), which can determine whether Advanced Glycosylated End Products (AGEs) antigen or antibody thereof is present or not in the sample based on the immunological chromatographic technique.

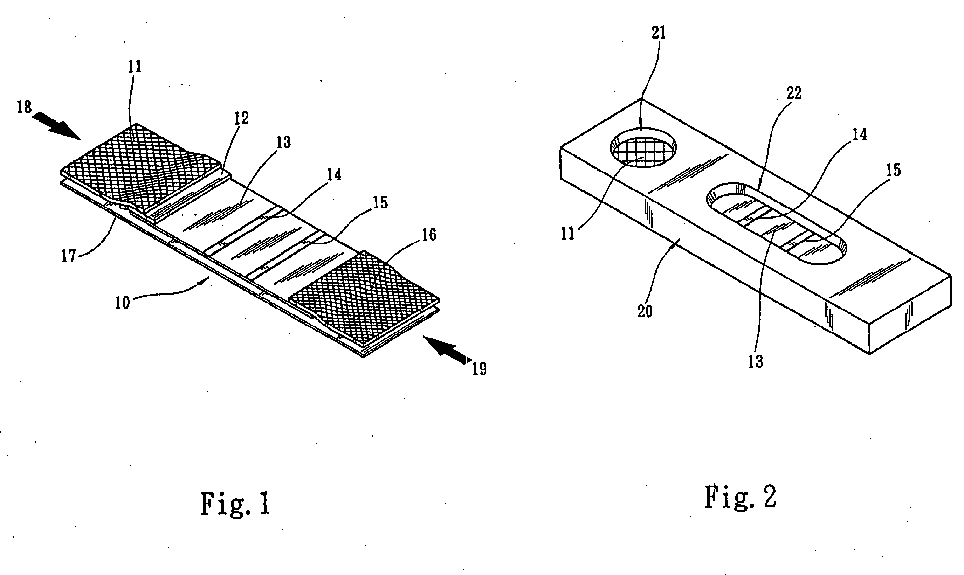

Referring to Figure 1, an immunological chromatographic test strip 10 comprises a porous fiber membrane 13 bonded on a base plate 17. A water absorption pad 11 for the sample is disposed at the most front end of the test strip 10, and is overlapped with the porous fiber membrane 13. A displaying carrier fiber block 12 is provided beneath the water absorption pad 11 for the sample and upon the porous fiber membrane 13 in a manner such that it is overlapped with them. The displaying carrier fiber block 12 has been impregnated with a blue displaying carrier and has an antibody or antigen immobilized thereon.

The color change against the reference line due to the formation of the immunological complex between, for example, the anti-glycosylated protein antibody and the glycosylated antigen (e.g., Advanced Glycosylated End Products (AGEs) antigen) on a base plate. A reading zone 14 which may be a spot or a line and which has antibody or antigen immobilized thereon is disposed on a section of the porous fiber membrane 13. The front end of the reading zone 14 faces the displaying carrier fiber block 12, while at a appropriate distance from its rear end, a reference zone 15 which may be a spot or a line is provided and which is also on a section of the porous fiber membrane 13 and which has antibody or antigen immobilized thereon. The reference zone 15 has its rear end facing an absorption pad 16. The material used to construct the above-mentioned porous fiber membrane 13 may be nylon fiber membrane, cellulose nitrate membrane, polyester fiber membrane, cellulose fiber membrane, synthetic fiber membrane and the like.

Referring to Figure 2, a waterproof device box 20 for accommodating the immunological test strip 10 is consisted of a sample port 21 and a port of the reaction zone 22.

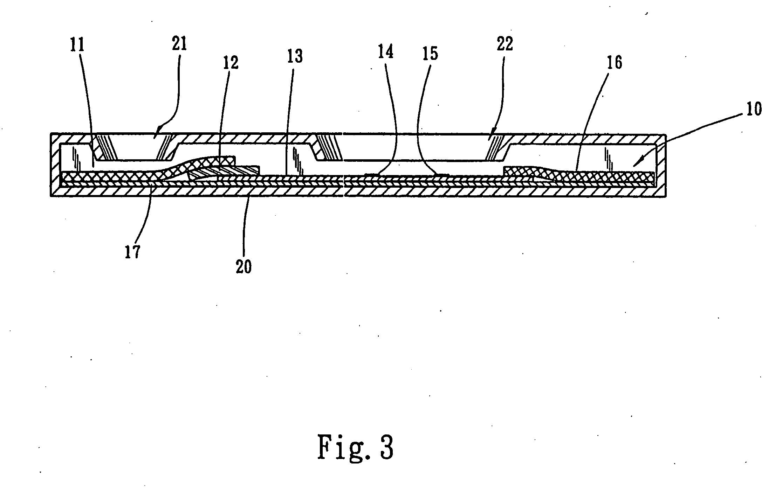

Figure 3 is a three-dimensional view of the waterproof device box 20 accommodating a test strip 10. Wherein, the water absorption pad for the sample 11 is just below the sample port 21, and contact therewith. The area of the sample port 21 should be less than that of the water absorption pad for the sample 11. The reading zone 14 and the reference zone 15 are disposed within the port of the reaction zone 22, but do not contact therewith for visualizing them directly. The absorption pad 16 is located at the rear end of the device box 20. Since, as described above, the blue displaying carrier on the displaying carrier fiber membrane 12 has antibody or antigen immobilized thereon, upon contacting the the blue displaying carrier on the displaying carrier fiber membrane 12 with the test sample, this blue displaying carrier will migrate freely on the test strip 10 in the direction toward the porous fiber membrane 13 and the absorption pad 16. The base plate 17 is associated on its lower side with the inner bottom side of the device box 20 and its upper side with the porous fiber membrane 13. The porous fiber membrane 13 is provided with a reading zone 14 and a reference zone 15, and has its voids filled fully with a blocking protein.

When a liquid test sample is added at the sample port 21, the test sample will react immediately with the antibody or antigen immobilized in the blue displaying carrier of the displaying carrier fiber block 12 and will migrate together with the blue displaying carrier toward the absorption pad 16. If a glycosylated protein antigen, e.g., a AGE antigen, is present in the test sample, it will react immediately with one or more antibodies of the glycosylated protein immobilized on the blue displaying carrier and occupy all of the epitope on the antibodies of the glycosylated protein. Since the binding sites of antibodies of the glycosylated protein immobilized on the blue displaying carrier will bind with the glycosylated protein antigen and are occupied thereby, they shall not bind with the one or more glycosylated protein antigen immobilized on the reading zone 14 such that all of the blue displaying carrier should pass the reading zone 14 and no visible blue line should appear on this zone. The blue displaying carrier will migrate further till bind with antibody or antigen (e.g., anti-rat immunoglobin G antibody) immobilized on the reference zone 15 to form a visible blue line. Therefore, a blue line on the reading zone 14 means a positive reaction, while a blue line will appear on the reference zone 15 in all cases disregarding a positive or negative reaction. On the other hand, if the test sample contains no target analyte, a portion of the one or more antibody against the glycosylated protein, will react with the glycosylated protein antigen, e.g., a AGE antigen, immobilized on the reading zone 14 and forms a visible blue line, while other portion of the blue displaying carrier will bind on the reference zone 15, i.e., a competitive reaction. Therefore, a blue line on the reading zone means a negative reaction. The absorption pad 16 is used for absorbing all of the liquid migrating to the end such that a capillary action can be sustained.

Alternatively, in the case as described above, a sandwich assay can be conducted just by replacing the glycosylated protein antigen immobilized on the reading zone 14 with one or more anti-glycosylated protein antibody.

The porous fiber membrane used in the invention has a pore size in the range of 0.1 - 60 micrometer. The displaying carrier used herein is a fine coloured particulate, fluorescent substance or enzyme. Such particulates may have a particle size of 0.01 - 20 micron and may be any of latex micro-particles, dye micro-particles, liposomes, colloidal gold particles, carbon black micro-particles, polymeric micro-particles. When the displaying carrier is a fine coloured micro-particle, the detecting result can be read directly and is referred as a direct displaying carrier. When the displaying carrier is an enzyme, the result can be read only after developing with a developing agent, and such displaying carrier is referred as indirect displaying carrier.

The base plate used in the invention is a waterproof plastic plate or waterproof paper. The materials used to construct the water absorption pad for the sample and the absorption pad used in the invention is not particularly limited, but it is better to have higher water absorbability. The displaying carrier fiber block is a water insoluble fibrous material.

The preferred embodiments are outlined in the claims.

The invention will be illustrated further more detailed with the following non-limiting examples. Only a device (see example 4) is claimed.

### Example 1

### Raising of the antibody of the glycosylated protein

The antibody of the glycosylated protein is prepared as a polyclonal antibody by immunizing directly a rabbit or a goat with purified antigen, e.g., AGE antigen, or by immunizing mice into hybridoma and further manipulating to yield as a monoclonal antibody.

### Example 2

### The agglutination assay of the glycosylated protein antigen

A polystyrene bead or other colored micro-particles having a particle size of about 0.8 micrometer was used as the displaying carrier micro-particle and was diluted into a concentration of 3%. The AGE antibody obtained in Example 1 above was diluted with a phosphate buffer into a concentration of 2 mg/ml. 10 ml each of the displaying carrier micro-particle suspension and the antibody solution were added into a glass tube and mixed well, which then stood for 8 hours. Thereafter, 1 g of bovine serum albumin (BSA) was added and mixed in the tube, which then stood for 8 hours. After centrifuging at 12000 rpm for 30 minutes, the supernatant was discarded, and repeated this operation once more. 2% BSA solution was added to a total amount of 20 ml. The mixture was sonicated into a homogeneous suspension as the desired displaying carrier suspension.

Three solutions of negative standard serum were prepared as having a AGE antigen content of 0, 1 and 2 units /ml, respectively. A solution of weak positive standard serum was prepared as having a AGE antigen content of 5 units /ml. A solution of strong positive standard serum was prepared as having a AGE antigen content of 16 units /ml. Finally, an unknown test sample was provided.

100 microliter each of the serum solutions prepared above was placed into a test device having the test sample loaded, respectively. 50 microliter of the displaying carrier suspension was added into each test device, and read results after allowing them developing for 3 - 5 minutes.

### Results:

| | The displaying carrier agglutination suspension |
|---|---|
| Negative standard serum 0 unit/ml | - |
| Negative standard serum 1 unit/ml | - |
| Negative standard serum 2.5 unit/ml | - |
| Weak positive standard serum 5 unit/ml | + |
| Strong positive standard serum 1-6 unit/ml | + |
| Unknown test sample | + |

An agglutination reaction means a positive reaction for the AGE antigen test, while no agglutination reaction means a negative reaction for the AGE antigen test. With the minimum positive reaction limit set at 5 unit/ml, the positive reaction of the unknown test sample indicates the concentration of the AGE antigen in this test sample is ≥ 5 unit/ml.

### Example 3

### Immunological turbidimetric assay of the glycosylated protein

0.2 g of the displaying carrier micro-particles was added into one liter of distilled water to prepare a suspension. The displaying carrier may be a white polystyrene bead having a particle size of about 0.3 micrometer. Alternatively, micro-particles having color at other wavelength can be employed also. To this suspension, 30 mg of the anti-AGE antibody was added and mixed well, and then stood for 18 hours. Next, 4 g of BSA was added and mixed well, and again stood for 18 hours. The mixture was centrifuged at 12000 rpm for 30 minutes and the resulting supernatant was discarded. This process was repeated three times. 2% BSA solution was added to a total amount of one liter. The mixture was sonicated into a homogeneous suspension as the desired displaying carrier suspension.

Six standard serum solutions were prepared as having a AGE antigen content of 0, 1, 2, 4, 8 and 16 units /ml, respectively. An unknown test sample was provided.

250 microliter of the displaying carrier suspension was added into a respective colorimetric tube. 20 micrometer each of the standard serum solution prepared above was added into each colorimetric tube, respectively. 20 microliter of the unknown test sample solution was added into another colorimetric tube.

The colorimeter was set to null with air at a wavelength of 340 nm. When 20 microliter each of the standard serum solution and unknown test sample were added, the OD value of individual colorimetric tube should read immediately and read again at 240 second thereafter. A OD difference of the reaction was calculated by subtracting the first OD value from the second OD value.

With the positive reaction limit set at ≥ 5 unit/ml, each reaction OD value obtained from each standard serum solution was plotted as a standard curve. The concentration of the AGE antigen in the test sample can then be calculated from this standard curve and the resulting value is 9 unit/ml, a positive reaction.

### Results:

| | OD value at 0 sec | OD value at 240 sec | Reaction OD value | Units |
|---|---|---|---|---|
| Standard serum A | 0.86 | 0.85 | 0.01 | 0 |
| Standard serum B | 0.92 | 0.87 | 0.05 | 1 |
| Standard serum C | 0.98 | 0.82 | 0.16 | 2 |
| Standard serum D | 1.02 | 0.83 | 0.19 | 4 |
| Standard serum E | 0.94 | 0.69 | 0.25 | 8 |
| Standard serum F | 0.99 | 0.69 | 0.3 | 16 |
| Unknown test sample G | 1.07 | 0.81 | 0.26 | 9 |

According to this example, negative serum solution having a concentration of 0 unit/ml and a positive serum solution having a concentration of 5 units/ml can be used to conduct a qualitative determination. If the reaction OD value of the unknown test sample is higher than the reaction OD value of the positive standard serum of 5 units/ml, a positive reaction is indicated. Otherwise, it is a negative reaction.

### Example 4

### Assay of the glycosylated protein antigen with the immunological test strip

A 3% suspension of blue displaying carrier micro-particles, which may be polystyrene beads, or other colored micro-particles having a particle size of about 0.3 micrometer, was prepared. An anti-AGE antibody was diluted with phosphate buffer into a concentration of 2 mg/ml. 10 ml each of these was placed in a glass tube and mixed well, and then stood for 8 hours. 1 g of BAS was then added and mixed well, and then stood for 8 hours. The mixture was centrifuged at 12000 rpm for 30 minutes and the resulting supernatant was discarded. This process was repeated two more. 2% of BSA, and 10% of sucrose solution were added to a total amount of 20 ml. The mixture was sonicated to form a homogeneous suspension as the desired displaying carrier suspension.

A displaying carrier fiber block strip of 0.4 x 4.5 cm was impregnated in this displaying carrier suspension containing 10% sucrose, removed and dried in a desiccators at room temperature. After drying, it was dried in a freeze-dryer for 2 hours and then packed in a sealed bag containing desiccant and stored at 4°C.

A pre-determined amount of the displaying carrier suspension was loaded on the water absorption pad or on the front end of the porous fiber membrane; however, the latter is less favor for mass production.

An anti-AGEs antibody (anti-AGEs Ab) solution was spray-coated and immobilized on a band at 1.8 cm of a piece of long cellulose nitrate film of 15 x 4.5 cm, which spray-coated line is referred as the reading zone. Then, a solution containing anti-rabbit IgG was spray-coated and immobilized on the band at 3.4 cm, which spray-coated line is referred as the reference line. Thereafter, the cellulose nitrate film was impregnated in a phosphate buffer containing 5% BAS for at least 2 hours. The cellulose nitrate film was removed, rinsed with fresh water, and dried in a desiccator at room temperature. Thereafter, the film was stuck and completely covered a plastic base plate starting from the 2 cm position. The film thus treated was then dried in a freeze-dryer for 2 hours and then stored in a sealed bag containing desiccant at 4°C.

A water absorption pad for the sample of 15 x 3 cm was provided. The material of the water absorption pad for the sample or the water absorption pad is not particularly limited, but it is better for its higher water absorbability, and its dimension is variable.

The carrier fiber block was interposed between the front end of the porous fiber membrane and the base plate in a manner that the carrier fiber block was overlapped with the cellulose nitrate film. Then, the absorption pad for the sample was stuck on the carrier fiber block and the base plate in a manner that it was overlapped with the carrier fiber block. Finally, the absorbing pad was stick over the rear end of the finished porous fiber membrane and on the end of the base plate. At least some area of the absorbing pad was overlapped with the fiber membrane. Test strips with a width of 0.5 cm was cut therefrom as the finished product.

The finished test strip was installed in a waterproof device box in a manner that the sample port of this device box is at a position just above the water absorption pad for the sample, and contact therewith. The area of the sample port should be less than that of the water absorption pad for the sample. While the reading zone and the reference zone on the test strip should be in a visible position within the reaction zone of the device box.

The water absorption pad for the sample is disposed in front of the reading zone. The displaying carrier fiber block was overlapped one another with the cellulose nitrate and the water absorption pad for the sample. The reference line was behind the reading zone and the water absorption pad was behind the reference line. At 5 minutes after adding the test sample, the result can be read directly with the eye viewing through the reaction zone.

150 microliter of the negative serum sample was added in the sample port, the blue displaying carrier will migrate toward the reaction line (the reading zone and the reference line) through the capillary principle, and reached finally the water absorption pad at the last end. Since this negative sample did not contain the AGEs antigen, the anti-AGE antibody immobilized on the blue displaying carrier would not react with the anti-AGE antibody immobilized on the reading zone and hence no visible blue line would form thereon. The blue displaying carrier would bind with the anti-rabbit IgG antibody immobilized on the reference line and form a visible blue line, which meant a negative reaction. The absorption pad would absorb all of the liquid migrated thereto such the capillary action could be sustained.

When 150 microliter of a positive serum sample was into the sample port, the anti-AGEs antibody immobilized on the blue displaying carrier would bind with the AGEs antigen contained in the serum sample before the blue displaying carrier reached the reading zone. At the time the blue displaying carrier reached the reading zone, the anti-AGEs antibody immobilized on the reading zone would bind again with the AGEs antigen and formed a visible blue line. The remaining blue displaying carrier would pass the reading zone, and bind on the reference line, which exhibited a sandwich positive reaction.

Alternatively, by contacting the front section of the finished test strip product with the test sample for a period of time, an experimental result similar to that described above could be obtained thereby.

As described above, other than the sandwich approach, the above experiment can be conducted by a competitive method. According to Example 4, the experiment can be accomplished through a competitive method just changing the substance immobilized on the reading zone, for example, the AGE protein antigen.

In conclusion, by testing with the specific reagent and the method according to the invention, the presence or not of the AGE in the diabetes can be readily known so that the practitioner can, at the earliest stage, prevent the progression of the complicated conditions in the diabetic patient or blocking the further progression of the complicated conditions.

## Claims

1. An immunological chromatographic analytical device for determining a glycosylated protein, wherein the glycosylated protein is an advanced glycosylated end product (AGE), which device comprises:
a test strip, comprising:
a base plate; and constitutive parts provided on said base plate, wherein said constitutive parts comprise:
(a) a water-absorption pad, provided at the front end of said test strip, with its rear end overlapped with the front end of a porous fiber membrane;
(b) a porous fiber membrane, provided on the base plate and having a reading zone thereon provided behind a displaying carrier fiber block;
(c) a displaying carrier fiber block, provided between the rear end of the water absorption pad for the sample and the front end of said porous fiber membrane, and being overlapped one another with these two parts; said displaying carrier fiber block having a displaying carrier loaded thereon; and said displaying carrier having affinity substances immobilized thereon; and
(d) at least one immobilized substance, disposed in the reading zone of said porous fiber membrane;
wherein, with a test strip constructed with the above-mentioned parts, after adding a test sample onto the water absorption pad for a period of time, the presence or not of the AGE antigen or anti-AGE antibody in said test sample can be determined based on the occurrence of a reaction color or not on the reading zone.

2. An immunological chromatographic analytical device as in claim 1, wherein the material of said porous fiber membrane is a nylon fiber membrane, a cellulose nitrate film, a polyester fiber membrane, a cellulose fiber membrane, or a polymeric fiber membrane.

3. An immunological chromatographic analytical device as in claim 1, wherein said pore size of said porous fiber membrane is in the range of about 0.1 micrometer to 60 micrometer.

4. An immunological chromatographic analytical device as in claim 1, wherein said displaying carrier is a colored micro-particle, an enzyme or a fluorescent substance.

5. An immunological chromatographic analytical device as in claim 1, wherein said micro-particle is a micro-particle selected from the group consisting of latex micro-particle, dye micro-particle, gold emulsion micro-particle, carbon black micro-particle, metal micro-particle, liposome, polymeric micro-particle.

6. An immunological chromatographic analytical device as in claim 1, wherein said displaying carrier has a particle size in the range of about 0.01 - 20 micrometer.

7. An immunological chromatographic analytical device as in claim 1, wherein said affinity substance is an AGE antigen having one structure.

8. An immunological chromatographic analytical device as in claim 1, wherein said affinity substance is an AGE antigen having various structures.

9. An immunological chromatographic analytic device as in claim 1, wherein said affinity substance is an anti-AGE antibody.

10. An immunological chromatographic analytical device as in claim 1, wherein said affinity substance is multiple anti-AGE antibodies.

11. An immunological chromatographic analytical device as in claim 1, wherein said immobilized substance is an AGE antigen having one structure.

12. An immunological chromatographic analytical device as in claim 1, wherein said immobilized substance is an AGE antigen having various structures.

13. An immunological chromatographic analytical device as in claim 1, wherein said immobilized substance is an anti-AGE antibody.

14. An immunological chromatographic analytical device as in claim 1, wherein said immobilized substance is multiple anti-AGE antibodies.

15. An immunological chromatographic analytical device as in claim 1, wherein said displaying carrier is a direct displaying carrier.

16. An immunological chromatographic analytical device as in claim 1, wherein said displaying carrier is an indirect displaying carrier.

17. An immunological chromatographic analytical device as in claim 1, comprising further a reference zone provided at an appropriate distance from said reading zone of said porous fiber membrane, wherein said reference zone has an antibody or antigen immobilized thereon.

18. An immunological chromatographic analytical device as in claim 1, useful for determining or quantitatively analyzing the result through optical method.

## Patentansprüche

1. Vorrichtung für die immunologische Chromatographieanalyse zur Bestimmung eines glycosylierten Proteins, wobei das glycosylierte Protein ein weiterentwickeltes glycosyliertes Endprodukt (AGE) ist, wobei die Vorrichtung folgendes aufweist:
einen Teststreifen, umfassend:
eine Grundplatte; und konstitutive Teile, die auf der Grundplatte vorgesehen sind, wobei diese konstitutiven Teile folgendes umfassen:
(a) ein Wasserabsorptionskissen, das am vorderen Ende des Teststreifens vorgesehen ist, wobei dessen hinteres Ende mit dem vorderen Ende einer porösen Fasermembran überlappt;
(b) eine poröse Fasermembran, die auf der Grundplatte vorgesehen ist und darauf eine Ablesezone aufweist, die hinter einem anzeigenden Trägerfaserblock vorgesehen ist;
(c) einen anzeigenden Trägerfaserblock, der zwischen dem hinteren Ende des Wasserabsorptionskissens für die Probe und dem vorderen Ende der porösen Fasermembran vorgesehen ist und gegenseitig mit diesen beiden Teilen überlappt ist; wobei der anzeigende Trägerfaserblock einen darauf aufgebrachten anzeigenden Träger aufweist; und wobei der anzeigende Träger darauf immobilisierte Affinitätssubstanzen aufweist; und
(d) zumindest eine immobilisierte Substanz, die sich in der Ablesezone der porösen Fasermembran befindet;
wobei bei einem Teststreifen, der mit den vorstehend genannten Teilen aufgebaut ist, nach der Zugabe einer Testprobe auf das Wasserabsorptionskissen für einen Zeitraum das Vorhandensein oder Nichtvorhandensein des AGE-Antigens oder AGE-Antikörpers in der Testprobe auf der Basis dessen bestimmt werden kann, ob eine Reaktionsfarbe auf der Ablesezone auftritt oder nicht.

2. Vorrichtung für die immunologische Chromatographieanalyse nach Anspruch 1, wobei das Material der porösen Fasermembran eine Nylonfasermembran, eine Cellulosenitratfolie, eine Polyesterfasermembran, eine Cellulosefasermembran oder eine Polymerfasermembran ist.

3. Vorrichtung für die immunologische Chromatographieanalyse nach Anspruch 1, wobei die Porengröße der porösen Fasermembran im Bereich von etwa 0,1 bis 60 µm liegt.

4. Vorrichtung für die immunologische Chromatographieanalyse nach Anspruch 1, wobei der anzeigende Träger ein gefärbtes Mikropartikel, ein Enzym oder eine fluoreszierende Substanz ist.

5. Vorrichtung für die immunologische Chromatographieanalyse nach Anspruch 1, wobei das Mikropartikel ein Mikropartikel ist, das aus der Gruppe ausgewählt ist, die aus einem Latexmikropartikel, Farbstoffmikropartikel, Goldemulsionsmikropartikel, Rußmikropartikel, Metallmikropartikel, Liposom, Polymermikropartikel besteht.

6. Vorrichtung für die immunologische Chromatographieanalyse nach Anspruch 1, wobei der anzeigende Träger eine Partikelgröße im Bereich von etwa 0,01 bis 20 µm aufweist.

7. Vorrichtung für die immunologische Chromatographieanalyse nach Anspruch 1, wobei die Affinitätssubstanz ein AGE-Antigen mit einer Struktur ist.

8. Vorrichtung für die immunologische Chromatographieanalyse nach Anspruch 1, wobei die Affinitätssubstanz ein AGE-Antigen mit verschiedenen Strukturen ist.

9. Vorrichtung für die immunologische Chromatographieanalyse nach Anspruch 1, wobei die Affinitätssubstanz ein AGE-Antikörper ist.

10. Vorrichtung für die immunologische Chromatographieanalyse nach Anspruch 1, wobei die Affinitätssubstanz mehrere AGE-Antikörper ist.

11. Vorrichtung für die immunologische Chromatographieanalyse nach Anspruch 1, wobei die immobilisierte Substanz ein AGE-Antigen mit einer Struktur ist.

12. Vorrichtung für die immunologische Chromatographieanalyse nach Anspruch 1, wobei die immobilisierte Substanz ein AGE-Antigen mit verschiedenen Strukturen ist.

13. Vorrichtung für die immunologische Chromatographieanalyse nach Anspruch 1, wobei die immobilisierte Substanz ein AGE-Antikörper ist.

14. Vorrichtung für die immunologische Chromatographieanalyse nach Anspruch 1, wobei die immobilisierte Substanz mehrere AGE-Antikörper ist.

15. Vorrichtung für die immunologische Chromatographieanalyse nach Anspruch 1, wobei der anzeigende Träger ein direkt anzeigender Träger ist.

16. Vorrichtung für die immunologische Chromatographieanalyse nach Anspruch 1, wobei der anzeigende Träger ein indirekt anzeigender Träger ist.

17. Vorrichtung für die immunologische Chromatographieanalyse nach Anspruch 1, die ferner eine Referenzzone umfaßt, die in einem geeigneten Abstand von der Ablesezone der porösen Fasermembran vorgesehen ist, wobei die Referenzzone einen Antikörper oder ein Antigen aufweist, der bzw. das darauf immobilisiert ist.

18. Vorrichtung für die immunologische Chromatographieanalyse nach Anspruch 1, die für die Bestimmung oder quantitative Analyse des Ergebnisses durch ein optisches Verfahren vorteilhaft ist.

## Revendications

1. Dispositif analytique chromatographique immunologique pour déterminer une protéine glycosylée, dans lequel la protéine glycosylée est un produit final glycosylé avancé (AGE), lequel dispositif comprend :
une bandelette d'essai, comprenant :
une plaque de base ; et des parties constitutives disposées sur ladite plaque de base, où lesdites parties constitutives comprennent :
(a) un tampon d'absorption de l'eau, disposé à l'extrémité avant de ladite bandelette, avec son extrémité arrière chevauchée par l'extrémité avant d'une membrane de fibre poreuse ;
(b) une membrane de fibre poreuse, disposée sur la plaque de base et comportant une zone de lecture au-dessus disposée à l'arrière d'un bloc de fibre à support d'affichage ;
(c) un bloc de fibre à support d'affichage, disposé entre l'extrémité arrière du tampon d'absorption de l'eau pour l'échantillon et l'extrémité avant de ladite membrane de fibre poreuse, et étant chevauchées l'une l'autre par ces deux parties ; un support d'affichage étant chargé sur ledit bloc de fibre à support d'affichage ; et des substances d'affinité étant immobilisées sur ledit support d'affichage ; et
(d) au moins une substance immobilisée, disposée dans la zone de lecture de ladite membrane de fibre poreuse ;
dans lequel, avec une bandelette d'essai construite avec les parties susmentionnées, après l'ajout d'un échantillon d'essai sur le tampon d'absorption de l'eau pendant une certaine période, on peut déterminer la présence ou non de l'antigène AGE ou de l'anticorps anti-AGE dans ledit échantillon d'essai d'après l'occurrence d'une couleur de réaction ou non sur la zone de lecture.

2. Dispositif analytique chromatographique immunologique selon la revendication 1, dans lequel la matière de ladite membrane de fibre poreuse est une membrane de fibre de nylon, un film de nitrate de cellulose, une membrane de fibre de polyester, une membrane de fibre de cellulose, ou une membrane de fibre polymère.

3. Dispositif analytique chromatographique immunologique selon la revendication 1, dans lequel ladite taille de pore de ladite membrane de fibre poreuse est dans la gamme d'environ 0,1 micromètre à 60 micromètres.

4. Dispositif analytique chromatographique immunologique selon la revendication 1, dans lequel ledit support d'affichage est une microparticule colorée, une enzyme ou une substance fluorescente.

5. Dispositif analytique chromatographique immunologique selon la revendication 1, dans lequel ladite microparticule est une microparticule choisie dans le groupe consistant en une microparticule de latex, une microparticule de teinte, une microparticule d'émulsion d'or, une microparticule de noir de carbone, une microparticule de métal, un liposome et une microparticule polymère.

6. Dispositif analytique chromatographique immunologique selon la revendication 1, dans lequel ledit support d'affichage a une taille de particule dans la gamme d'environ 0,01 à 20 micromètres.

7. Dispositif analytique chromatographique immunologique selon la revendication 1, dans lequel ladite substance d'affinité est un antigène AGE ayant une seule structure.

8. Dispositif analytique chromatographique immunologique selon la revendication 1, dans lequel ladite substance d'affinité est un antigène AGE ayant diverses structures.

9. Dispositif analytique chromatographique immunologique selon la revendication 1, dans lequel ladite substance d'affinité est un anticorps anti-AGE.

10. Dispositif analytique chromatographique immunologique selon la revendication 1, dans lequel ladite substance d'affinité est constituée de multiples anticorps anti-AGE.

11. Dispositif analytique chromatographique immunologique selon la revendication 1, dans lequel ladite substance immobilisée est un antigène AGE ayant une seule structure.

12. Dispositif analytique chromatographique immunologique selon la revendication 1, dans lequel ladite substance immobilisée est un antigène AGE ayant diverses structures.

13. Dispositif analytique chromatographique immunologique selon la revendication 1, dans lequel ladite substance immobilisée est un anticorps anti-AGE.

14. Dispositif analytique chromatographique immunologique selon la revendication 1, dans lequel ladite substance immobilisée est constituée de multiples anticorps anti-AGE.

15. Dispositif analytique chromatographique immunologique selon la revendication 1, dans lequel ledit support d'affichage est un support d'affichage direct.

16. Dispositif analytique chromatographique immunologique selon la revendication 1, dans lequel ledit support d'affichage est un support d'affichage indirect.

17. Dispositif analytique chromatographique immunologique selon la revendication 1, comprenant en outre une zone de référence disposée à une distance appropriée de ladite zone de lecture de ladite membrane de fibre poreuse, dans lequel un anticorps ou antigène est immobilisé sur ladite zone de référence.

18. Dispositif analytique chromatographique immunologique selon la revendication 1, utile pour déterminer ou analyser quantitativement le résultat par une méthode optique.
